# EUROPEAN PATENT APPLICATION

(11) **EP 1 797 824 A1**
(43) Date of publication of application: **20.06.2007**
(21) Application number: 05425891.8
(22) Date of filing: 16.12.2005
(51) Int. Cl.: A61B 17/04, A61B 17/22, A61B 17/30, A61B 1/313

(54) **Laparoscopy surgical device**

(71) Applicant: Universita' degli studi di Bari, 70121 Bari (IT)
(72) Inventor: Puglisi, Francesco, 70126 Bari (IT)
(74) Representative: Gislon, Gabriele

(57) **Abstract**

A surgical device for retrieving needles or fragments thereof from the abdominal cavity during laparoscopy operations, comprising a handle (1), a portion (2) that can be extended from said handle and sliding therein and a terminal (3) engaging the needle or fragment thereof, which is provided with magnetic means (4) for temporarily holding said needle or fragment.

## Description

The present invention relates to a device for laparoscopy or laparotomy surgery.

More particularly, the invention relates to a surgical device for retrieving needles from the abdominal cavity.

Laparoscopy is a surgical technique that consists in introducing gas in the abdominal cavity to obtain the stretching of the same, and subsequently performing short cuts on the abdomen through which dedicated surgical instruments and a micro-camera recording the operative area are introduced. Due to the presence of gas, the introduction of the instruments is carried out through particular cannulae inserted in the abdomen wall, which are known per se in the art as "trocar". These cannulae are provided with valve devices which prevent that the gas may escape from the abdominal cavity when the instruments are being either introduced or withdrawn.

Accordingly, in laparoscopy, trocars have to provide a seal to prevent that the carbon dioxide fed into the abdominal cavity escapes and to allow maintaining a suitable intra-abdominal gas pressure.

The surgical instruments are handled from the outside by means of handles which are out of the abdominal cavity; between the handle and the terminal portion of the instrument, there extends an instrument portion having an elongated cylinder shape which allows the same to be sealingly introduced through the trocar valves located at the incisions.

Tissue suture is one of the steps in common with many laparoscopic operations. To the purpose, there is introduced a metal needle connected to a wire, which are made of different materials and calibre according to the requirements. The needle is directed and manoeuvred by means of a needle forceps, i.e. an instrument provided with jaws to be clamped for gripping the needle. During the suture step, the needle with the forceps is forced through the tissue to be sutured until it protrudes from the other side, and then it is gripped at the protruding portion either by the same forceps or a different forceps.

After the suture has been completed, the wire is cut and withdrawn from the abdominal cavity together with the needle.

A problem with the suture step is that the needle may get lost, which may occur either in this step, or in the subsequent step when the needle is being withdrawn from the abdominal wall through the above-mentioned valves.

After the needle has been lost, i.e. it is no longer clamped between the jaws of the needle forceps, it may be very difficult to retrieve. In fact, the micro-camera shooting range may not be sufficiently wide to locate the needle, and the needle may slip between the intestinal loops and the intra-abdominal fatty tissue, away from said shooting range. Therefore, the difficulty of retrieving the needle by the needle forceps adds to the difficulty of finding the same.

The technique currently used for retrieving the needle provides that an X-ray is carried out in an intraoperative manner for locating the needle, which is radio-opaque. This aid is, however, not always sufficient for retrieving the needle, since the X-ray only provides a bidimensional image of the involved area, whereby any evaluation of the needle depth location is so difficult that the abdomen sometimes has to be opened for investigation.

The problems discussed above with reference to losing the needle also occur when the needle breaks, and a fragment of the needle gets lost. In this case, retrieving the fragment is even more difficult due to the small size thereof.

The object of the present invention is to solve the above-mentioned drawbacks, by providing a device for safely retrieving a lost needle, or fragments thereof, in the abdomen cavity, without having to open the abdomen or protracting the laparoscopy incisions, and however with a saving of time.

This object is achieved by means of the present invention which provides a surgical device for retrieving needles from the abdominal cavity, characterized in that it comprises a handle, a portion extending from said handle and a terminal for engaging the needle or fragment thereof, said terminal being provided with magnetic engaging means for holding said needle or needle fragment.

As will be detailed below, the handle is hold fixed within the trocar and the portion extending, i.e. sliding, within the handle is manoeuvred by the user.

According to a preferred embodiment, the magnetic terminal has a concave engaging face and the recess is sized such as to house the needle within said cavity. For example, the recess is substantially semi-circular with a radius greater than the needle radius.

The device according to the invention has a number of advantages as compared with the prior art.

In fact, it allows for a safe grip and facilitates withdrawing the needle, or needle fragment, from the abdominal cavity, it can be used in the presence of the sealing trocar valves, it can be easily washed and sterilized, and avoids having to open the abdomen in case of laparoscopy operations, since it can be introduced through the operating trocars. In addition, the device according to the invention is radio-opaque and its operation can be optionally controlled by means of radioscopy, such as illustrated in Fig. 7, discussed below.

A further advantage is that, by using the magnet, the device only requires to be approached to the needle to be able to "grip" the latter by means of the magnetic field, which is strong enough to attract the needle even from a distance of 1-3 cm. To retrieve the needle, accordingly, it is sufficient to approach the same either visually or by means of X-ray investigations. This permits to avoid having to locate the needle in a very precise manner in the three dimensions, since the magnet is able to attract the needle and hold the same even at a distance of 1-3 centimetres.

The invention will be now described in greater detail with reference to the annexed drawings, which are intended as illustrative rather than limiting, where:
- Fig. 1 is a side view of the device according to the invention;
- Fig. 2 is a side view of the device from Fig. 1, in the expanded form;
- Fig. 3 is an enlarged side view of the terminal and the magnetic engaging means of the device of the above figures;
- Fig. 4 is an enlarged bottom plan view of a further embodiment of the end and magnetic engaging means of the inventive device;
- Fig. 5 is a sectional schematic view of a different embodiment of the device of Fig. 4;
- Fig. 6 is a sectional view of the magnetic terminal from Fig. 4; and
- Fig. 7 is a facsimile of the image obtained by means of radioscopy when the inventive device is being used.

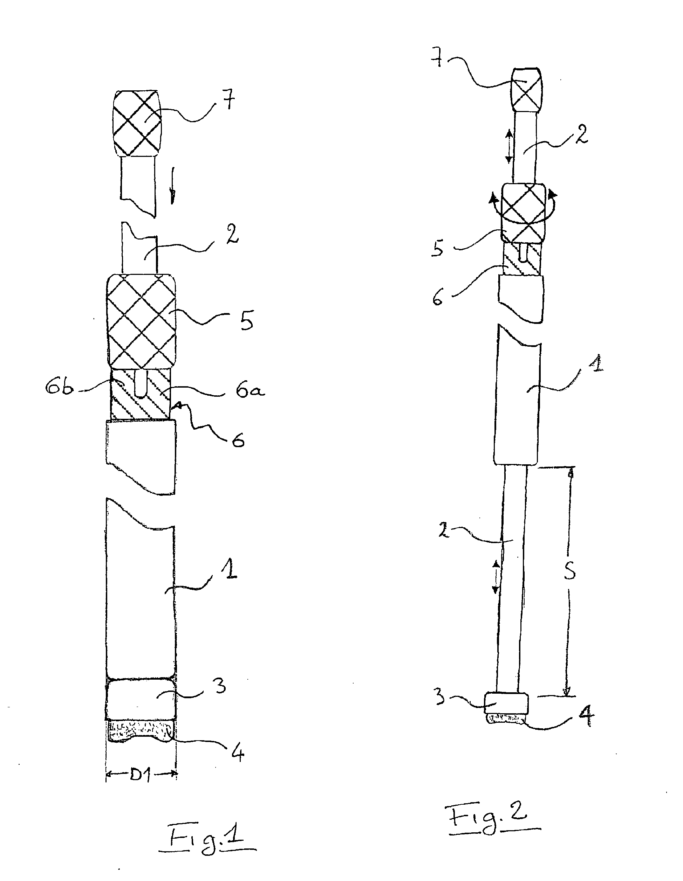

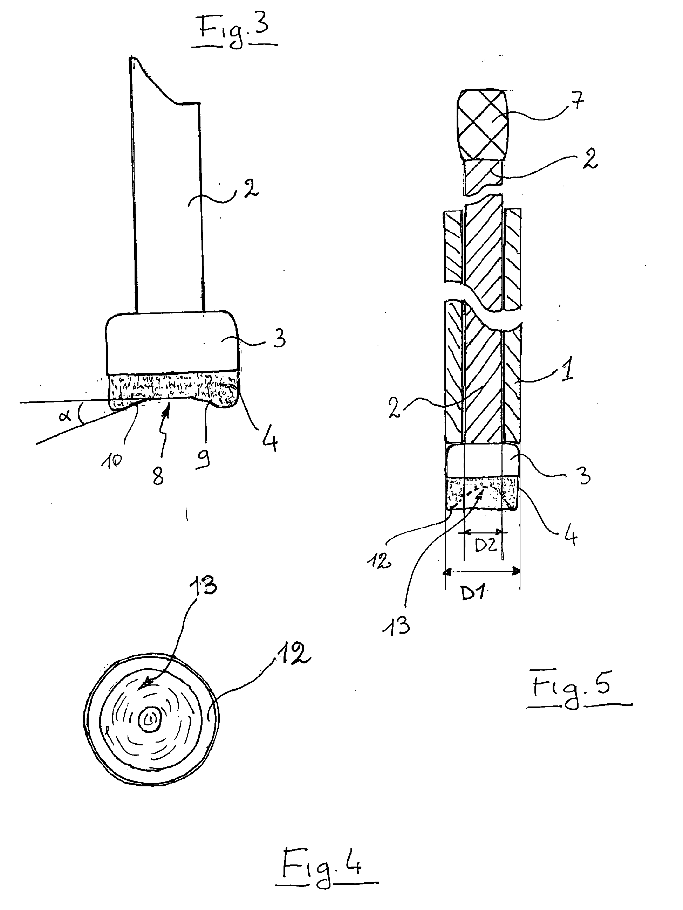

Referring first to Fig. 1, the device according to the invention comprises a substantially cylindrical handle 1, a rod portion 2 which is partially housed within the handle 1 and which can be extended from said handle and a terminal 3 for engaging the needle or a fragment thereof. The terminal 3 is provided with magnetic engaging means 4 for temporary holding said needle or fragment, i.e. for magnetically engaging and holding the needle until the same is gripped and/or removed from the abdominal cavity. For clarity, only the word "needle" will be used in the description below, but this term is intended to include also a fragment thereof or a similar object to be retrieved.

As may be seen in the figures, the terminal 3 has a circular cross section with diameter D1 substantially equal to, or lower than, the diameter of the handle 1, and in any case such as to pass through the trocar valve inserted in the patient's abdominal wall.

The magnetic engaging means 4 have, in turn, a diameter substantially equal to the diameter of the terminal 3, such that stagnation of organic material is prevented as much as possible, and cleaning and sterilizing the device are facilitated.

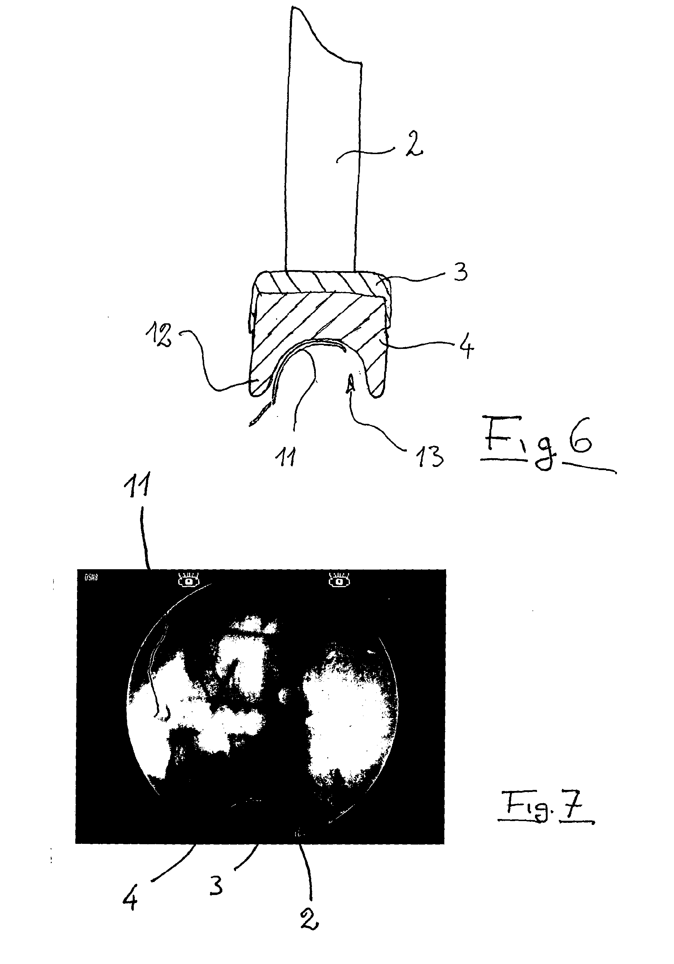

The terminal 3 is integral with a rod 2, which, as seen above, telescopically extends from the handle 1, in which it slides. In other words, the device portion consisting of the rod 2 at which end there is fixed the terminal 3 carrying the magnet 4 is movable from a first position where it is adjacent to the handle 1 and possibly in contact thereto (Fig. 1) to a second position, shown in Fig. 2, where the terminal 3 is spaced apart from the handle 1.

The second position is selected among a plurality of possible positions as a function of the distance S required between the terminal 3 and the handle 1 for being able to grip the needle. In fact, when the device is being used, the handle 1 is sealingly positioned in the trocar valve, where it is hold fixed to prevent gas leakage, white the position of the terminal is changed so to allow for reaching the needle: this option is particularly useful since the needle search is carried out in three dimensions, whereas the result of the radioscopy carried out to locate the same only provides "bidimensional" information, the depth of the needle being unknown.

For this reason, it must be possible to change the position of the terminal in a continuous manner without moving the handle 1 within the trocar, which must be fixed to provide the required gas seal. By adopting a fixed handle and a rod sliding therein and carrying the terminal 3 and magnet 4 at the end thereof, the desired result is achieved.

This step of removing the lost needle is shown in Fig. 7. This radioscopic image shows the terminal portion of the device according to the invention, which is perfectly visible, since it is totally radio-opaque, in the right lower part of the photograph; the rod 2, terminal 3 and magnet 4 are clearly visible. The needle 11 can be seen on the right side of the image.

The embodiment shown in Fig. 1 and 2 comprises a nut 5 that can be screwed on a clamping element 6 of the rod 2. The clamping element 6 is integral with the handle 1 and has two or more tongues 6a and 6b, as it is known in the art, which are pressed against the rod 2 when the nut 5 is screwed on the element 6 by moving the latter towards the handle 1. On the contrary, the rod, which can be caused to slide within the handle for reaching the desired position, is unlocked by unscrewing the nut 5.

Alternatively, Fig. 5, the clamping means 5, 6, 6a and 6b are not provided and the rod 2 slides within the handle 1. As may be seen in Fig. 5, the diameter D2 of the rod 2 is slightly lower than the inner diameter of handle 1, such that a controlled sliding of the rod within the handle is obtained though being able to operate the device with one hand which holds the handle in position and causes the rod to slide within the handle.

In the embodiment as shown in the figures, there is an individual rod 2 being formed as one piece that extends through the handle 1 and can be caused to slide therethrough. In a further embodiment, not illustrated, the rod 2 can consist of two (or more) elements that are telescopically engaged to each other to obtain a compact basic configuration, in which the rod portions are inserted within each other.

A nut 7 is screwed at the end of the rod 2 in order to prevent the latter from accidentally sliding off the handle 1; the nut 7 is removed after use such that the rod 2 can be removed from the handle and the device can be cleaned and sterilized.

As may be seen in Fig. 3 and 4, the engaging face of the magnetic means 4 is concave. In the embodiment from Fig. 3, the engaging face comprises a middle portion 8 which is planar or substantially planar and two side portions 9 and 10 which are planar or substantially planar and inclined towards said middle portion 8. In Fig. 3 there is shown the angle α as formed by the planes of the middle portion 8 and side portion 10, the side portion 9 being substantially symmetrical to the side portion 10 relative to the middle portion 8.

The angle α is preferably comprised within the range between 10 and 30 degrees.

Fig. 4, 5 and 6 show a plan and cross view of the device terminal being provided with the needle engaging magnet. In this embodiment, the magnet is concave and has a recess 13 that is circumferentially defined by an edge 12 such as to be capable of internally housing a curved needle 11 (Fig. 6). The shape of the recess 13 is, for example, substantially semi-spherical, with a radius greater than the radius of the needle 11. With this embodiment a safe grip of the needle is ensured, because the latter does not contact the surrounding tissues when it is being withdrawn from the abdominal cavity since it is housed in the recess. Thereby, the needle is prevented from damaging said tissues or being lost upon the withdrawal step, which can thus be carried out with the device according to the invention, i.e. without gripping the retrieved needle by means of needle forceps.

The magnetic means 4 are selected from permanent magnets and electromagnets. Particularly, simple or double permanent magnets will be used. In an embodiment, the magnet is covered with ferromagnetic material, such as steel, for improved cleaning and sterilization.

Fig. 7 shows the device according to the invention during an operation of withdrawing a needle 11 from an abdominal cavity during a laparoscopy operation. In the image, there is shown below on the right the device comprising the rod 2, the terminal 3 and the magnet 4 while moving towards a needle 11 which is positioned on the left of the image. As mentioned above, and as may be observed in the image in Fig. 7, the device according to the invention can be inserted within the abdominal cavity through a trocar valve and its position can be immediately visualized by means of radioscopy; the surgeon can thus move the terminal 3 towards the needle 11 and attract the same by means of the magnet 4 also in a remote manner. The needle 11 is thus housed within the cavity 13 of the magnet engaging face, where it is protected from any contact with the surrounding tissues by means of the edge 12 of said face of the magnet 4. The same edge 12 also prevents that the needle may contact the trocar when the needle is being withdrawn from the abdominal cavity, this withdrawal being necessarily carried out through a trocar; thereby the risk of losing again the needle during said withdrawal is avoided.

## Claims

1. A surgical device for retrieving needles or fragments thereof from the abdominal cavity, **characterized in that** it comprises a handle (1), a portion (2) that can be extended from said handle and a terminal (3) for engaging the needle or fragment thereof, said terminal being provided with magnetic engaging means (4) for temporarily holding said needle or fragment.

2. The device according to claim 1, wherein said portion (2) extending from said handle (1) is a rod sliding within said handle and projecting from both ends of said handle.

3. The device according to claim 1 or 2, wherein said magnetic engaging means (4) has a concave engaging face.

4. The device according to claim 3, wherein said concave face has a recess (13) that is defined by an edge (12) and is capable of housing a curved needle (11).

5. The device according to claim 3 or 4, wherein said concave face comprises a planar central portion (8) and one or more side portions (9, 10) which are planar and inclined towards said central portion (8).

6. The device according to claim 5, wherein the angle (α) between the planes of said inclined side portions (9, 10) and the plane of said middle portion (8) is comprised within the range between 10 and 30 degrees.

7. The device according to one of preceding claims, wherein said magnetic engaging means comprise a permanent magnet.

8. The device according to claim 7, further comprising a coating of ferromagnetic material for said permanent magnet.
